# EUROPÄISCHE PATENTSCHRIFT

(11) **EP 1 504 733 B1**
(45) Veröffentlichungstag und Bekanntmachung des Hinweises auf die Patenterteilung: **06.06.2007**
(21) Anmeldenummer: 03017769.5
(22) Anmeldetag: 04.08.2003
(51) Int. Cl.: A61F 2/46, A61F 2/00, A61F 2/44

(54) **Zervikalprothese mit Einsetzinstrument**
Cervical prosthesis with insertion instrument
Prothèse cervicale avec instrument d'insertion

(43) Veröffentlichungstag der Anmeldung: 09.02.2005
(73) Patentinhaber: Cervitech Inc., Rockway, NJ 07866 (US)
(72) Erfinder: Keller, Arnold, 23863 Kayhude (DE)
(74) Vertreter: Glawe, Delfs, Moll

(56) Entgegenhaltungen:
- WO-A-01/06962
- WO-A-03/026522
- WO-A-03/037228
- FR-A- 2 795 945

## Beschreibung

Es ist bekannt (EP-A 333990), Zwischenwirbelprothesen mittels eines Einsetzinstruments zu implantieren, das ein Paar von Greiforganen aufweist, die die Prothese an gegenüberliegenden Seiten fassen. Die starr miteinander verbundenen Greiforgane enthalten je eine in AP-Richtung verlaufende Nut zur Aufnahme der beiden in AP-Richtung verlaufenden Plattenränder, die darin durch Reibung oder durch eine federnde Klinke gehalten sind. Wenn die Prothese die ihr zugedachte Implantationsstellung erreicht hat, wird das Instrument von ihr abgezogen. Dabei gleiten die Plattenränder in AP-Richtung aus den Nuten heraus. Derartige Greiforgane sind nur für Endplatten geeignet, deren Seitenränder parallel zueinander verlaufen und einen vorbestimmten, stets gleichen Abstand voneinander haben. Sie sind ungeeignet für kleine Prothesen, wie sie im Zervikalbereich verwendet werden, sowie für solche Prothesen, die keinen Seitenrand haben, der zum Zusammenwirken mit den Greiforganen ausgebildet ist. Schließlich sind sie auch nicht geeignet für Prothesen, die sehr genau positioniert werden sollen und deren Lage relativ zum Einsetzinstrument nicht durch die beim Implantieren auftretenden Kräfte zufällig verändert werden darf. WO 03/037228 zeigt eine Anordnung entsprechend dem Oberbegriff des Anspruchs 1 (siehe insbesondere Abbildungen 69 bis 74).

Der Erfindung liegt die Aufgabe zugrunde, die Implantation einer Zervikalprothese unter Verwendung eines Einsetzinstruments sicherer zu machen.

Die Erfindungsgemäß Lösung besteht in den Merkmalen des Anspruchs 1.

Die Erfindung bedient sich einer Anordnung, die aus einer Zervikalprothese und einem Einsetzinstrument besteht, dessen Greiforgane zum Greifen der Prothese auf gegenüber liegenden Seiten einander genähert und voneinander entfernt werden können. Die Prothese und die Greiforgane sind mit komplementären Vorsprüngen und Ausnehmungen versehen, die in AP-Richtung der Prothese formschlüssig positionsbestimmend sind. Solange die Greiforgane um die Prothese geschlossen sind, kann sie - anders als bei der zuvor beschriebenen bekannten Anordnung - ihre Stellung relativ zum Einsetzinstrument nicht in derjenigen Ebene ändern, die zur Erstreckung der Prothesenplatten parallel ist. Sie können also auch nicht in AP-Richtung aus den Greiforganen herausrutschen. Allerdings ist es nicht ganz einfach, die Prothesenteile an dem Einsetzinstrument zu montieren, weil die Vorsprünge und Ausnehmungen sehr klein sind. Auch kann es leicht geschehen, daß eine Fehlmontage nicht auffällt.

Ferner ist ein Halter vorgesehen, der die Prothese paßgenau aufnimmt. Dadurch ist gewährleistet, daß die Vorsprünge bzw. Ausnehmungen an der Prothese sich an vorbestimmter Stelle des Halters befinden. Der Halter ist an derjenigen Seite, die der anterioren Seite der Prothese entspricht, zur Entnahme der Prothese offen. Er weist Führungsorgane auf, durch die die Greiforgane des Einsetzinstruments in eine Stellung relativ zur Prothese geführt werden, in welcher sich die komplementären Vorsprünge und Ausnehmungen an der Prothese und an den Greiforgane fluchtend und eingriffsbereit gegenüberstehen. Wenn der Operateur aus dieser Stellung heraus die Greiforgane schließt, kommen die Vorsprünge und Ausnehmungen mit Sicherheit in Eingriff miteinander, und die erstrebte, korrekte Montage der Prothese am Einsetzinstrument kommt sicher zustande.

Damit die Prothese die paßgenaue Position im Halter einnimmt und beibehält, sind zweckmäßigerweise lösbare Mittel zum Fixieren der Prothese in dieser Stellung am Halter vorgesehen. Die Lösbarkeit dieser Mittel kann darauf beruhen, daß sie elastisch nachgiebig sein, wenn die am Einsetzinstrument montierte Prothese aus dem Halter herausgezogen wird. Sie kann auch darauf beruhen, daß sie oder ihre Befestigung am Halter bei der Entnahme der Prothese zerstört werden. Es kann sich auch um einen Deckel handeln der die Prothese im Halter festlegt und zu ihrer Entnahme geöffnet wird. Schließlich besteht auch die Möglichkeit, den Halter als ganzes zu zerstören, indem er beispielsweise mit Sollbruchstellen oder Aufreißlinien versehen wird. Bei dem Halter kann es sich um einen Wegwerfartikel handeln.

Die Erfindung wird im folgenden näher unter Bezugnahme auf die Zeichnungen erläutert, die ein vorteilhaftes Ausführungsbeispiel schematisch darstellt. Es zeigen:
- Fig. 1: die Gesamtansicht eines Einsetzinstruments mit Zervikalprothese
- Fig. 2: die Prothese perspektivisch im größeren Maßstab,
- Fig. 3: das Greiforgan des Einsetzinstruments,
- Fig. 4: den Halter mit darin befindlicher Prothese im Schnitt parallel zur Prothesenebene,
- Fig. 5: einen Schnitt durch den Halter längs der Sagittalebene der darin befindlichen Prothese und
- Fig. 6: eine Ventralansicht des Halters mit darin befindlicher Prothese und geschnitten erscheinenden Greiforganen.

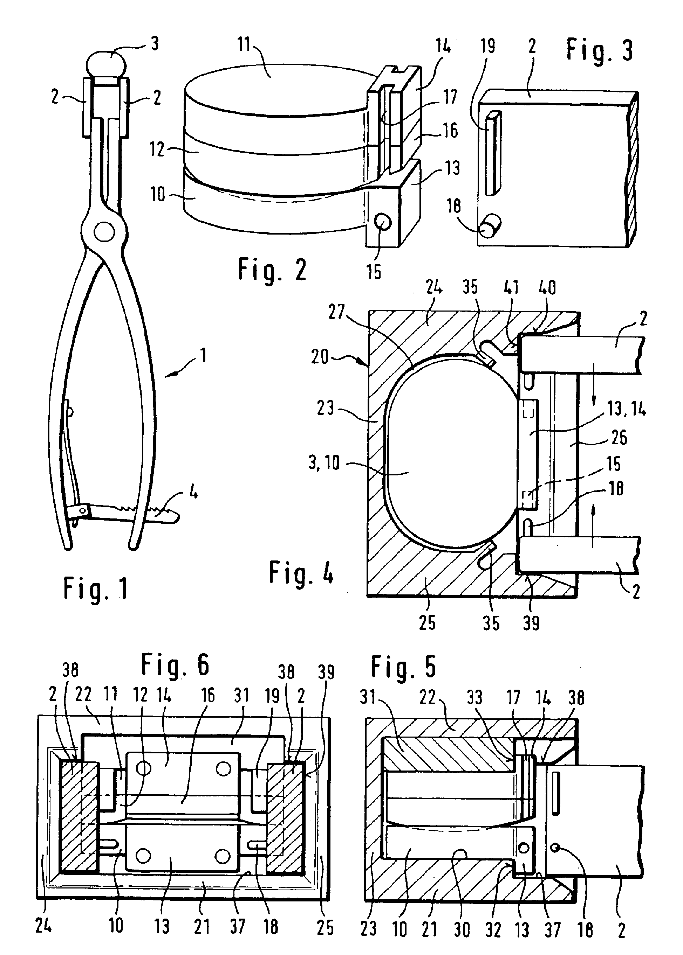

Das Einsetzinstrument 1 ist als Zange ausgebildet, die am vorderen Ende ein Paar von Greiforganen 2 aufweist, die die Prothese 3 von entgegengesetzten Seiten her faßt. Die AP-Richtung (anterior-posterior) stimmt im dargestellten Beispiel mit der Längsrichtung des Einsetzinstruments überein. Dies ist zweckmäßig. Jedoch kann die Längsrichtung des Instruments auch ein wenig von der AP-Richtung der Prothese abweichen. In Fig. 2 ist die dorsale Seite der Prothese links angeordnet. Die Zange kann in der geschlossenen Stellung durch eine lösbare Sperre 4 gesichert werden.

Gemäß Fig. 2 besteht die Prothese aus zwei Abschlußplatten 10, 11 und einem Prothesenkern 12, der mit der Abschlußplatte 10 ein Gelenk bildet. Die Höhe der Prothese ist variabel, nämlich durch Verwendung unterschiedlich hoher Prothesenkerne 12. Die Abschlußplatten 10, 11 weisen entlang ihrem ventralen Rand je einen Flansch 13, 14 auf, der etwas schmaler ist als die plattenförmig ausgedehnten Teile der Abschlußplatten 10, 11 und des Prothesenkerns 12. Der Flansch 13 der Abschlußplatte 10 enthält in seinen Seitenflächen je eine Bohrung 15. Der Flansch 14 der Abschlußplatte 11 sowie der entsprechende Teil 16 des Prothesenkerns 12 enthalten in ihren Seitenflächen einen lotrecht zur Plattenerstreckung verlaufenden Schlitz 17. Beide Seiten sind symmetrisch gleich ausgebildet.

Jeder Bohrung 15 entspricht einem Stift 18 und jedem Schlitz 17 entspricht eine vorspringende Leiste 19 an den Greiforganen 2. Der Grund für die Wahl eines Schlitzes 17 an den Teilen 11, 12 liegt darin, daß der Prothesenkern 12 unterschiedliche Höhe haben kann. Die Leiste 19 kann mit Schlitzen 17 von Teilen 11, 12 unterschiedlicher Höhe zusammenwirken.

Der Halter 20 besteht aus einer unteren Wand 21, einer oberen Wand 22, einer Rückwand 23 und Seitenwänden 24, 25. An der Vorderseite 26 ist er offen. Die Wände umgrenzen eine Raum 27 zur Aufnahme der Prothese 3. Seine Höhe kann genau der Höhe einer Prothese entsprechen. Damit derselbe Haltertyp für unterschiedliche hohe Prothesen verwendet werden kann, ist es zweckmäßiger, wenn seine lichte Höhe zwischen den Innenflächen 30 der unteren Wand 21 und der oberen Wand 22 etwas größer als die Höhe der Prothesen ist und der verbleibende Raum durch ein Füllstück 31 geschlossen wird, das eine zur jeweiligen Prothesenhöhe passende Dicke hat. Die Höhenposition der Prothese im Halter ist jedenfalls eindeutig festgelegt.

In LM-Richtung (lateral-medial) wird die Position der Prothese im Halter durch die Seitenwände 24, 25 bestimmt.

Die untere Wand 21 und das Füllstück 31 haben eine Stirnflächen 32, 33, die Anschlagflächen für die dorsalen Seiten der Flanschen 13, 14 bilden und dadurch in der AP -Richtung die Position der Prothese im Halter 20 bestimmen. Festgehalten wird die Prothese in dieser Position durch Federzungen 35 an den Seitenwänden 24, 25, die als nachgiebige Fixiermittel für die Prothese im Halter dienen. Ihre Kraft ist so bemessen, daß die Prothese die eindeutige Stellung relativ zum Halter unter den bei Transport, Lagerung und Handhabung auftretenden Kräften mit Sicherheit nicht verläßt. Der Operateur kann sich daher darauf verlassen, daß die Prothese diese Stellung innerhalb des Halters einnimmt, wenn er sie mit den Einsetzinstrument 1 verbinden will.

Die Öffnung 26 des Halters 20 wird durch untere und obere Führungsflächen 37, 38 sowie seitliche Führungsflächen 39, 40 umgrenzt. Diese Flächen enden an Stirnflächen 41, die mit den Anschlagflächen 32, 33 zusammenfallen können. Der Abstand der oberen und unteren Öffnungsflächen 37, 38 gleicht mit ein wenig Spiel der Höhe der Greiforgane 2 des Einsetzinstruments. Die Weite zwischen den seitlichen Öffnungsflächen 39, 40 gleicht der Weite des Instruments in einem hinreichend weit geöffneten Zustand. Die Flächen 37 bis 41 sind daher dazu geeignet, die Greiforgane 2 des Einsetzinstruments vor der Verbindung mit der Prothese zu positionieren. Die Maße sind so gewählt, daß in dieser Position die Vorsprünge 18, 19 der Greiforgane und die Ausnehmungen 15, 17 der Prothese einander genau fluchtend, eingriffsbereit gegenüberstehen. Werden die Greiforgane 2 des Einsetzinstruments aus dieser Position in Richtung der in Fig. 4 angedeuteten Pfeile zusammengeführt, so gelangen die Vorsprünge und Ausnehmungen mit Sicherheit in korrekten Eingriff miteinander. Die geschlossene Stellung der Greiforgane wird durch die Rastsperre 4 gesichert. Der Halter kann nun von der im Einsetzinstrument gehaltenen Prothese 3 unter elastischer Verformung der Zungen 35 abgezogen werden.

## Patentansprüche

1. Anordnung umfassend eine Zervikalprothese (3) und ein Einsetzinstrument (1), das ein Paar von die Prothese (3) auf gegenüberliegenden Seiten fassenden Greiforganen (2) aufweist, wobei die Greiforgane (2) und die Prothese (3) komplementäre Vorsprünge (18, 19) und Ausnehmungen (15, 17) aufweisen, die die Relativposition der Prothese (3) zumindest in deren AP-Richtung eindeutig bestimmten und durch Relativbewegung der Greiforgange (2) in Richtung quer zur Längsrichtung des Einsetzinstruments (1) und quer zur AP-Richtung der Prothese (3) in Eingriff zu bringen und voneinander zu lösen sind, **dadurch gekennzeichnet, daß** die Anordnung einen Halter (20) umfaßt, der die Prothese (3) paßgenau aufnimmt und auf der anterioren Seite der Prothese (3) eine Öffnung (26) mit Führungsorganen (37 bis 41) zum Führen des Einsetzinstruments (1) in eine Entnahmestellung aufweist, in der sich die komplementären Vorsprünge (18, 19) und Ausnehmungen (15, 17) eingriffsbereit gegenüber stehen.

2. Anordnung nach Anspruch 1, **dadurch gekennzeichnet, daß** lösbare Mittel (35) zum Fixieren der Prothese (3) in der paßgenauen Stellung im Halter vorgesehen sind.

## Claims

1. Arrangement comprising a cervical prosthesis (3) and an insertion instrument (1) which has a pair of gripping members (2) that grip the prosthesis (3) on opposite sides, the gripping members (2) and the prosthesis (3) having complementary projections (18, 19) and recesses (15, 17) which precisely define the relative position of the prosthesis (3), at least in its AP direction, and which are designed to be brought into engagement and to be released from one another by relative movement of the gripping members (2) in a direction transverse to the longitudinal direction of the insertion instrument (1) and transverse to the AP direction of the prosthesis (3), **characterized in that** the arrangement comprises a holder (20) which receives the prosthesis (3) with an exact fit and which, on the anterior face of the prosthesis (3), has an opening (26) with guide elements (37 to 41) for guiding the insertion instrument (1) into a removal position in which the complementary projections (18, 19) and recesses (15, 17) lie opposite one another ready for engagement.

2. Arrangement according to Claim 1, **characterized in that** releasable means (35) are provided for fixing the prosthesis (3) in the holder in the exact-fit position.

## Revendications

1. Ensemble comprenant une prothèse cervicale (3) et un instrument d'insertion (1), qui comporte une paire d'organes de saisie (2) saisissant la prothèse (3) sur des côtés opposés, dans lequel les organes de saisie (2) et la prothèse (3) présentent des saillies (18, 19) et des cavités (15, 17) complémentaires, qui déterminent nettement la position relative de la prothèse (3) au moins dans sa direction AP et qui peuvent être engagées les unes dans les autres et séparées les unes des autres par un mouvement relatif de organes de saisie (2) dans une direction transversale à la direction longitudinale de l'instrument d'insertion (1) et transversale à la direction AP de la prothèse (3), **caractérisé en ce que** l'ensemble comprend un support (20), qui contient la prothèse (3) en ajustage précis et présente sur la face antérieure de la prothèse (3) une ouverture (26) avec des organes de guidage (37 à 41) pour guider l'instrument d'insertion (1) dans une position d'enlèvement, dans laquelle les saillies (18, 19) et les cavités (15, 17) complémentaires se trouvent en face les unes des autres prêtes à un engagement.

2. Ensemble selon la revendication 1, **caractérisé en ce qu'**il est prévu des moyens séparables (35) pour fixer la prothèse (3) dans la position ajustée avec précision dans le support.
